# EUROPEAN PATENT SPECIFICATION

(11) **EP 3 095 774 B1**
(45) Date of publication and mention of the grant of the patent: **03.07.2019**
(21) Application number: 15168940.3
(22) Date of filing: 22.05.2015
(51) Int. Cl.: C07C 29/20, C07C 45/00, B01J 8/06

(54) **PROCESS FOR THE PRODUCTION OF A MIXTURE COMPRISING CYCLOHEXANONE AND CYCLOHEXANOL**
VERFAHREN ZUR HERSTELLUNG EINES GEMISCHES MIT CYCLOHEXANON UND CYCLOHEXANOL
PROCÉDÉ POUR LA PRODUCTION D'UN MÉLANGE COMPRENANT DU CYCLOHEXANONE ET CYCLOHEXANOL

(43) Date of publication of application: 23.11.2016
(73) Proprietor: CAP III B.V., 6129 EL Urmond (NL)
(72) Inventor: TINGE, Johan Thomas, 6129 EL Urmond (NL); MARTENS, Wilhelmus Rudolf Maria, 6129 EL Urmond (NL)
(74) Representative: Cohausz & Florack

(56) References cited:
- WO-A1-2009/080621
- WO-A1-2011/073233
- US-A- 4 108 912
- US-A- 5 484 576

## Description

The present invention relates to a continuous process for the production of a mixture comprising cyclohexanone and cyclohexanol by hydrogenation of phenol, which process is performed in an industrial scale chemical plant comprising a multi-tubular reactor according to claim 1; to the use of an industrial scale chemical plant comprising a multi-tubular reactor according to claim 12; and to a process for constructing such an industrial scale plant according to claim 14.

Cyclohexanone is an intermediate in the production of, amongst other compounds, adipic acid and caprolactam. These are monomers commonly used in the production of polyamide-6,6 and polyamide-6, respectively. A major process for the production of cyclohexanone for use in producing caprolactam is based on selective oxidation of cyclohexane, using atmospheric oxygen. Oxidation of cyclohexane yields a mixture of cyclohexanol and cyclohexanone and the precursor cyclohexyl hydroperoxide which is then thermally and/or catalytically decomposed to produce additional cyclohexanol and cyclohexanone, and a variety of by-products. Cyclohexanone may be separated by distillation from the mixture comprising cyclohexanol, cyclohexanone, unreacted cyclohexane and by-products. Cyclohexanol may also be recovered by distillation and optionally converted to cyclohexanone by dehydrogenation. Chemical processes for the production of cyclohexanone and cyclohexanol by oxidation of cyclohexane are known in the art (see for example Michael Tuttle Musser; Cyclohexanol and Cyclohexanone in Ullmann's Encyclopedia of Industrial Chemistry Published Online: 15 OCT 2011 DOI: 10.1002/14356007.a08_217.pub2 Copyright © 2002 by Wiley-VCH Verlag GmbH & Co. KGaA) (Musser).

Typically, cyclohexane is produced from hydrogenation of benzene. Hydrogenation of benzene to cyclohexane can be carried out in the vapour phase (also often called: gas phase) or in the liquid phase, in the presence of catalytic material and hydrogen. In general, in these hydrogenation processes a heterogeneous catalyst is used, for example Raney nickel or a supported catalyst with nickel, palladium, or platinum as catalytic material. The hydrogenation of benzene to cyclohexane is known to be highly exothermic. High temperatures can lead to thermodynamic limitations on benzene conversion, thermal cracking and increased by-product formation and because of that the reactor temperature is generally controlled to be in the range of from 150 to 350°C. Present industrial processes for the production of cyclohexane through the catalytic hydrogenation of benzene differ principally in their methods for the removal of the heat of reaction. This is done by indirect cooling with a liquid in a jacketed reactor; or by vaporization of feed and recycle of product in a vapour phase, or by a multi-reactor process with intercooling between reactors. Still another process uses a catalyst slurry with a liquid pump-around for indirect cooling. In order to produce high purity cyclohexane product (99.5% or higher) the effluent of the first benzene hydrogenation reactor is often, optionally after cooling, fed to a second benzene hydrogenation reactor.

Typically, the first benzene hydrogenation reactor is a (vertical) multi-tubular catalytic reactor with catalytic material arranged in the tubes and a coolant which circulates externally around the tubes, that is fed with a gaseous mixture comprising hydrogen and benzene. In this first reactor the vast majority of the fed benzene is converted into cyclohexane. Heat of reaction is removed by indirect cooling with a coolant. In general, the coolant is a liquid, which is optionally evaporated. In case liquid water is applied as coolant then steam is optionally obtained by evaporation of liquid water within the shell of the reactor. As used herein, a coolant is a heat carrier that is used for cooling purposes.

One alternative process for the production of cyclohexanone is by the catalytic reduction of phenol with hydrogen, for example using a palladium-comprising heterogeneous catalyst. The reduction of phenol with hydrogen can be performed in the vapour phase (also often called: gas phase) or in the liquid phase, described in, for example, Musser. The hydrogenation of phenol is known to be highly exothermic. A mixture comprising cyclohexanol, cyclohexanone, unreacted phenol and by-products is produced. Separation of cyclohexanone from this mixture may be made by distillation. Cyclohexanol may also be recovered by distillation and optionally converted to cyclohexanone by dehydrogenation. Unreacted phenol is removed by distillation and recycled in the process.

Typically, a phenol hydrogenation reactor is a (vertical) multi-tubular catalytic reactor with catalytic material in the tubes and a coolant which circulates externally around the tubes, that is fed with a gaseous mixture comprising hydrogen and phenol. In this reactor the vast majority of the fed phenol is converted into cyclohexanone. Heat of reaction is removed by indirect cooling with a coolant. In general, the coolant is a liquid, which is optionally evaporated. In case liquid water is applied as coolant then steam is optionally obtained by evaporation of liquid water within the shell of the reactor. The reactor temperature is generally controlled to be in the range of from 100 to 250°C.

Chemical plants for the production of a mixture comprising cyclohexanone and cyclohexanol by reduction of phenol, and chemical plants for the production of cyclohexane by reduction of benzene are known in the art, also described in, for example, Musser. The starting materials, intermediates, products, by-products and catalysts used in such a plant for the production of a mixture comprising cyclohexanone and cyclohexanol based on hydrogenation of phenol are vastly different to those used in a plant for the production of cyclohexane based on hydrogenation of benzene. Accordingly, the apparatus required for the hydrogenation of phenol is different to that required for the hydrogenation of benzene.

WO 2011/073233 A1 refers to a method for hydrogenating an aromatic compound and in particular to a method for preparing cyclohexanone, cyclohexanol or a mixture thereof in a continuous way by catalytically hydrogenating phenol fed into a reactor comprising a supported hydrogenation catalyst, comprising a dopant selected from the group of alkali metal hydroxides, alkaline earth metal hydroxides, alkaline earth metal oxides, carbonates of alkali metals and carbonates of alkaline earth metals, and in which process during the hydrogenation of phenol continuously or intermittently water is fed into the reactor, the weight to weight ratio of water fed into the reactor to phenol fed into the reactor on average being 0.1 or less.

Nowadays, due to severe reduction of the phenol price compared to the benzene price, the process for the production of a mixture comprising cyclohexanone and cyclohexanol by reduction of phenol with hydrogen is more economic regarding raw materials costs and energy costs than the process for producing a mixture comprising cyclohexanone and cyclohexanol by the hydrogenation of benzene followed by the oxidation of the obtained cyclohexane. So, for current producers of mixtures of cyclohexanone and cyclohexanol that apply the process of hydrogenation of benzene followed by the oxidation of the obtained cyclohexane it is advantageous to switch to the process of producing a mixture comprising cyclohexanone and cyclohexanol by hydrogenation of phenol. An option for switching to the process based on the hydrogenation of phenol is to build a new plant. A major drawback of this approach are the high investment costs for such a new plant. In particular a reactor suitable for the hydrogenation of phenol is typically complex and therefore expensive.

The present inventors have discovered a method to significantly reduce the investment costs of a plant for the production of a mixture comprising cyclohexanone and cyclohexanol based on the reduction of phenol. They have developed a process for the production of a mixture comprising cyclohexanone and cyclohexanol by hydrogenation of phenol; based on the equipment of a chemical plant used to produce a mixture comprising cyclohexane from the hydrogenation of benzene. More specifically, a continuous process for the production of a mixture comprising cyclohexanone and cyclohexanol by hydrogenation of phenol according to claim 1, the use of an industrial scale chemical plant in the process according to claim 1, the use of an industrial scale chemical plant according to claim 12 and to a process for the construction of an industrial scale chemical plant for the production of a mixture comprising cyclohexanone and cyclohexanol by hydrogenation of phenol according to claim 14.

In particular the invention concerns a continuous process for the production of a mixture comprising cyclohexanone and cyclohexanol by hydrogenation of phenol, which process is performed in an industrial scale chemical plant comprising a multi-tubular reactor characterised in that said multi-tubular reactor has been used for the hydrogenation of benzene; wherein the benzene hydrogenation catalyst inside the tubes of the multi-tubular reactor has been replaced with phenol hydrogenation catalyst; and wherein "has been used" means that the multi-tubular reactor was designed for the hydrogenation of benzene and installed in a plant for the hydrogenation of benzene.

Such a process is useful to a company which operates a plant for the hydrogenation of benzene to cyclohexane, who wishes to discontinue use of such plant It is also useful to a company who wishes to construct a plant for the production of a mixture comprising cyclohexanone and cyclohexanol.

The invention further relates to the use of an industrial scale chemical plant for the production of a mixture comprising cyclohexanone and cyclohexanol by hydrogenation of phenol, which chemical plant comprises a multi-tubular characterised in that said multi-tubular reactor has been used for the hydrogenation of benzene; wherein the benzene hydrogenation catalyst inside the tubes of the multi-tubular reactor has been replaced with phenol hydrogenation catalyst; and wherein "has been used" means that the multi-tubular reactor was designed for the hydrogenation of benzene and installed in a plant for the hydrogenation of benzene.

As used herein, "has been used" includes that the multi-tubular reactor was designed for the hydrogenation of benzene and/or installed in a plant for the hydrogenation of benzene.

Processes based on oxidation of cyclohexane to produce essentially pure cyclohexanone are known to consume large amounts of energy, typically supplied industrially as steam and electricity. Specific steam consumptions of more than 5 tons of steam per ton of purified cyclohexanone are known. Large quantities of steam are consumed in heating cyclohexane fed to the (first) oxidation reactor; the removal of unreacted cyclohexane, distillative purification of cyclohexane and the dehydrogenation of cyclohexanol. A further advantage of a process of constructing a plant to carry out a different process according to the present invention is that the energy consumption per unit weight of cyclohexanone produced may be reduced.

Typical carbon efficiency of hydrogenation of phenol to cyclohexanone is higher than 98 % and in general even higher than 99 %, while the carbon efficiency of oxidation of cyclohexane to cyclohexanone is typically from 75 % to 90 %. A yet further advantage of operating a plant according to the present invention is that less starting material is required to produce a given amount of cyclohexanone. Further, the amount of by-products and therefore waste produced per unit weight of cyclohexanone produced may be reduced.

The production of cyclohexanone by the oxidation of cyclohexane is typically subject to stringent safety regulations because of the risk of ignition of explosive cyclohexane-oxygen mixtures, for example a large safety circle must be in place. Yet, a further advantage of the process of the present invention is that the risk of explosion of cyclohexane-oxygen mixtures is avoided, because no cyclohexane is used in the process of the plant for the hydrogenation of phenol. Thus the associated safety measures are not required.

Also disclosed herein is a chemical plant suitable for the production of a mixture comprising cyclohexanone and cyclohexanol by hydrogenation of phenol, which chemical plant comprises a multi-tubular reactor characterised in that said multi-tubular reactor has been used for the hydrogenation of benzene.

Typically, in the process of the present invention said chemical plant for the hydrogenation of phenol comprises one or more of the following units: a partial condenser unit suitable for condensing cyclohexanol and cyclohexanone from the gaseous mixture comprising cyclohexanone and cyclohexanol, a distillation column suitable for distilling overhead components having a lower boiling point than cyclohexanone, a distillation column suitable for the separation of cyclohexanone with a purity of more than 99 wt.%, a distillation column suitable for distilling overhead components having a higher boiling point than cyclohexanone, a cyclohexanol hydrogenation reactor, wherein cyclohexanol is converted into cyclohexanone and hydrogen.

Typically, in the chemical plant of the present invention the chemical plant further comprises a distillation column suitable for the separation of cyclohexanone from a mixture of cyclohexanone and cyclohexanol with a purity of more than 99 wt.%.

Disclosed herein is cyclohexanone prepared in a chemical plant, in which chemical plant a mixture comprising cyclohexanone and cyclohexanol is produced through a continuous process for the hydrogenation of phenol, which chemical plant comprises a multi-tubular reactor characterised in that said multi-tubular reactor has been used for the hydrogenation of benzene.

A chemical plant includes all apparatus necessary to manufacture or otherwise process the chemicals desired. This includes units for one or multiple chemical or physical operations, for example, heating up, cooling down, mixing, distillation, extraction and reaction. It includes all auxiliary equipment, for example reflux units, coolant supply, pumps, heat exchangers and pipework. The exact apparatus depends amongst others on type and purity of the starting material(s) and the desired end product(s), but also on the scale and type of process.

By a continuous process for the hydrogenation of phenol is meant a process in which phenol and hydrogen are fed without interruption and whereby a hydrogenated product or a mixture of hydrogenated products of phenol are withdrawn without interruption. A continuous process for the hydrogenation of phenol may be at a constant rate or may fluctuate in rate over time. A continuous process for the hydrogenation of phenol may be interrupted for a certain period of time due to e.g. a process disturbance, a maintenance activity, or for economic reasons.

The chemical plant is preferably of industrial scale. By industrial scale is meant a hydrogenation rate of phenol of at least 1,000 kg of phenol per hour; more preferably, at least 2,000 kg of phenol hour; even more preferably, at least 4,000 kg of phenol per hour; and most preferably, at least 6,000 kg of phenol hour.

By hydrogenation of benzene is meant that benzene and hydrogen are partly or completely converted and whereby a hydrogenated product or a mixture of hydrogenated products of benzene are formed.

As used herein, a mixture comprising cyclohexanone and cyclohexanol that is produced in a process for the hydrogenation of phenol is the mixture of compounds resulting from the hydrogenation of phenol. Typically, this comprises cyclohexanone, cyclohexanol, at least one characteristic by-product and (unreacted) phenol. The phenol content of this mixture is typically at least 0.02 wt.%. Preferably, it is at least 0.03 wt.%; more preferably, at least 0.1 wt.%. The phenol content is preferably, less than 50 wt.%; more preferably, less than 20 wt.%; most preferably, less than 10 wt.%. The mixture is typically gaseous.

Typically, the mixture comprising cyclohexanone and cyclohexanol that is produced comprises one or more of the following components: phenol, 2-phenylcyclohexanol, 3-phenylcyclohexanol, 4-phenylcyclohexanol, cyclohexyl phenylether, benzofuran, 2,3-dimethylbenzofuran, 3-methyl-4-octanone, 4-methyl-3-octanone, 3-methyl-3-octanone, methyl-isopropylcyclohexanol, methyl-isopropylcyclohexanone and 1-(4-methylpentane-2-yl)-benzene-phenol. In addition , the gaseous mixture comprising cyclohexanone and cyclohexanol that is produced might also comprise (unconverted) hydrogen and inert components like nitrogen and methane.

Typically, in the process of the present invention the mixture comprising cyclohexanone and cyclohexanol that is produced also comprises phenol and at least one compound selected from 2-phenylcyclohexanol, 3-phenylcyclohexanol, 4-phenylcyclohexanol, cyclohexyl phenylether, benzofuran, 2,3-dimethylbenzofuran, 3-methyl-4-octanone, 4-methyl-3-octanone, 3-methyl-3-octanone, methyl-isopropylcyclohexanol, methyl-isopropylcyclohexanone and 1-(4-methylpentane-2-yl)-benzene-phenol.

Typically, in the process of the present invention the molar ratio of cyclohexanone to cyclohexanol in the mixture comprising cyclohexanone and cyclohexanol that is produced is more than 4 to 1. Preferably, the molar ratio of cyclohexanone to cyclohexanol in the mixture comprising cyclohexanone and cyclohexanol that is produced is more than 6 to 1. More preferably, the molar ratio is more than 10 to 1; yet more preferably, the molar ratio is more than 15 to 1.

Typically, in the process of the present invention cyclohexanone is separated from the mixture comprising cyclohexanone and cyclohexanol that is produced. More preferably, cyclohexanone with a purity of more than 90 wt.% is separated; even more preferably, cyclohexanone with a purity of more than 98 wt.% is separated. Typically, the separation of cyclohexanone is performed by distillation, crystallization, extraction and/or a combination thereof.

Typically, in the process of the present invention from the mixture comprising cyclohexanone and cyclohexanol that is produced, cyclohexanone with a purity of more than 99 wt.% is separated by distillation.

Typically, the hydrogenation of phenol whereby a mixture comprising cyclohexanone and cyclohexanol is produced is performed in a chemical plant comprising a multi-tubular reactor. The number of reactor tubes in the multi-tubular reactor is typically more than 5. Preferably, it is more than 10. More preferably, it is more than 25. The number of reactor tubes is typically less than 100,000. Preferably, it is less than 50,000. More preferably, it is less than 20,000.

Typically, in the process of the present invention the number of reactor tubes in said multi-tubular reactor is from 25 to 20,000.

The internal diameter of the shell of the multi-tubular reactor is typically more than 50 mm. Preferably, it is more than 100 mm. More preferably, it is more than 200 mm. The internal diameter of the shell of the multi-tubular reactor is typically less than 10 m. Preferably, it is less than 8 m. More preferably, it is less than 6 m.

Typically, in the process of the present invention the internal diameter of the shell of said multi-tubular reactor is from 0.2 to 6 m.

The internal diameter of the reactor tubes is typically more than 2 mm. Preferably, it is more than 5 mm. More preferably, it is more than 10 mm. The internal diameter of the reactor tubes is typically less than 500 mm. Preferably, it is less than 250 mm. More preferably, it is less than 120 mm.

Typically, in the process of the present invention the internal diameter of the reactor tubes in said multi-tubular reactor is from 10 to 120 mm. Preferably, all reactor tubes in the multi-tubular reactor have (almost) the same internal diameter.

In the tubes in the multi-tubular reactor heat is generated due to hydrogenation of phenol, thereby heating up the mixture of components in the tubes. Heat of the mixture of components is removed by indirect cooling with a coolant. The temperature of the mixture comprising cyclohexanone and cyclohexanol that is discharged from the multi-tubular reactor is typically more than 60 °C. Preferably, it is more than 80 °C. More preferably, it is more than 100 °C. The temperature of the mixture comprising cyclohexanone and cyclohexanol that is discharged from the multi-tubular reactor is typically less than 260 °C. Preferably, it is less than 240 °C. More preferably, it is less than 220 °C.

Typically, in the process of the present invention the temperature of the mixture comprising cyclohexanone and cyclohexanol that is discharged from the multi-tubular reactor is from 100 to 220 °C.

Typically, in a multi-tubular reactor wherein phenol is hydrogenated heat is removed by a coolant. Usually, as coolant aqueous or organic solvents or mixtures thereof are applied. Typically, in the process of the present invention water is applied as coolant in the multi-tubular reactor.

By absorbing heat the coolant that is charged to the multi-tubular reactor is heated up, evaporated (partly or completely) or a combination thereof. Preferably, at least 10 wt.% of the coolant that is charged to the multi-tubular reactor is evaporated. More preferably, at least 50 wt.%. Even more preferably, at least 90 wt.%.

Typically, in the process of the present invention water is applied as coolant in the multi-tubular reactor and more than 90 wt.% of the water evaporated.

The hydrogenation of phenol with hydrogen can be performed in the gas phase or in the liquid phase. Preferably, the hydrogenation of phenol with hydrogen is performed in the gas phase. Typically, a process for the hydrogenation of phenol that is performed in the gas phase is charged with a gaseous mixture comprising the raw materials phenol and hydrogen as feed. Preferably, the phenol content of the mixture comprising the raw materials phenol and hydrogen that is charged as feed to the multi-tubular reactor is at least 5 wt.%. More preferably, at least 15 wt.%. Even more preferably, at least 25 wt.%.

Typically, in the process of the present invention a mixture comprising phenol and hydrogen, in which the phenol content is more than 25 wt.%, is charged as feed to the multi-tubular reactor.

The hydrogenation catalyst may in principle be any (supported) hydrogenation catalyst capable of catalysing the hydrogenation of phenol. Usually, the catalyst comprises one or more catalytically active metals selected from palladium, platinum, ruthenium, rhodium, iridium, rubidium and osmium. Palladium, platinum or a combination thereof are preferred catalytically active metals.

Typically, in the process of the present invention the hydrogenation is carried out in the presence of a supported palladium catalyst.

The support may in principle be any support capable of supporting one or more catalytically active metals for the hydrogenation of phenol. Suitable supports in particular may be selected from the group of alumina, activated carbon, titanium oxide, calcium carbonate and carbon black. Another support that may be used is silica.

Typically, in the process of the present invention the hydrogenation is carried out in the presence of a supported palladium catalyst.

In general, the per pass conversion of phenol in the reaction unit is more than 90 %. Optionally, unreacted hydrogen gas and inerts are separated off from the reaction mixture. Usually, unreacted hydrogen gas is re-used in the phenol hydrogenation process.

As used herein, the meaning of an "essentially pure" component is that the content of the component is at least 98 wt.%. Preferably, it is at least 99 wt.%; more preferably, at least 99.5 wt.%; even more preferably, at least 99.9 wt.%.

FIG 1 shows a plant for the production of cyclohexane.

FIG 2 shows a plant according to the present invention, for the production of cyclohexanone.

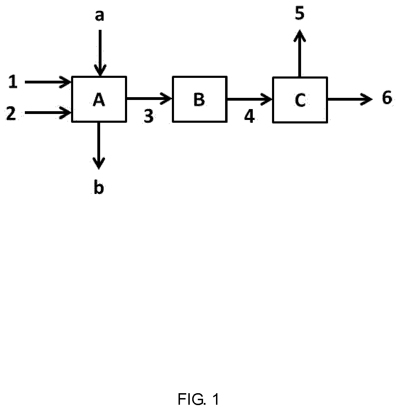

FIG 1 shows a plant for the production of cyclohexane by first hydrogenating benzene in the vapour phase to form a hydrogenated mixture and then separating a hydrogen-comprising flow from the hydrogenated mixture. The benzene hydrogenation is carried out in a two-reactors-in-series process with as first hydrogenation reactor a multi-tubular reactor.

Gaseous benzene is fed to the first hydrogenation unit [A] through line [1]. The first hydrogenation unit [A] comprises one or more multi-tubular reactors with benzene hydrogenation catalyst inside the tubes and indirect cooling with a coolant. Coolant is fed through line [a] and after being heated discharged through line [b]. Hydrogen gas is fed through line [2]. The resulting gaseous first hydrogenated mixture which comprises cyclohexane and unconverted benzene is fed through line [3] to the second hydrogenation unit [B]. Optionally, this mixture is cooled before being fed to the second hydrogenation unit [B] (not shown in FIG. 1). Optionally, additional hydrogen is fed to the second hydrogenation unit [B] (not shown in FIG. 1). In the second hydrogenation unit [B] unconverted benzene is hydrogenated into cyclohexane. The second hydrogenation unit [B] comprises one or more benzene hydrogenation reactors. Optionally, the second hydrogenation unit [B] is cooled (not shown in FIG 1). The gaseous second hydrogenated mixture is removed to hydrogen separation unit [C] through line [4]. From hydrogen separation unit [C] a gaseous hydrogen-comprising flow and a cyclohexane-comprising flow are separately discharged through line [5] and line [6], respectively. Hydrogen separation unit [C] might comprise a partial condensation unit and one or more distillation units. Optionally, the gaseous hydrogen-comprising flow discharged through line [5] is fed to the first hydrogenation unit [A] (not shown in FIG. 1).

Optionally, the cyclohexane discharged through line [6] is fed to cyclohexanone plant based on oxidation of cyclohexane (not shown in FIG 1).

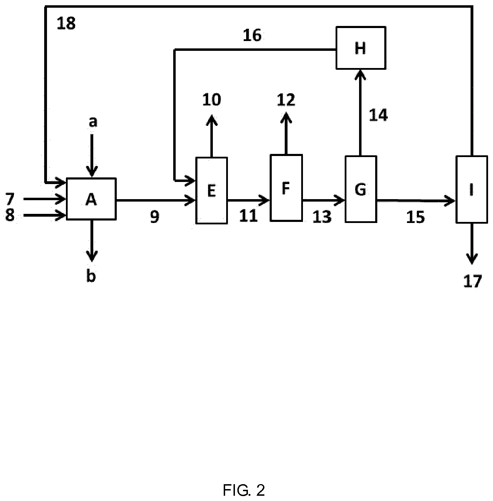

FIG. 2 shows a plant according to the present invention, for the production of cyclohexanone by first hydrogenating phenol in the vapour phase, then separating cyclohexanone from the resulting mixture comprising cyclohexanol and cyclohexanone and finally dehydrogenating cyclohexanol into cyclohexanone.

Fresh phenol is fed via line [7], hydrogen gas is fed through line [8] and a mixture comprising phenol that is distilled overhead in phenol distillation column [I] is fed through line [18] to first hydrogenation unit [A]. First hydrogenation unit [A] comprises one or more multi-tubular reactors with phenol hydrogenation catalyst inside the tubes and indirect cooling with a coolant. Coolant is fed through line [a] and after being heated discharged through line [b]. The resulting mixture of reaction products, comprising phenol, cyclohexanol and cyclohexanone is fed through line [9] to lights distillation column [E]. Optionally, unreacted hydrogen gas and inert gases are separated from this mixture (not shown in FIG. 2). A mixture of components with boiling points below that of cyclohexanone is distilled overhead and removed through line [10]. The bottom product is fed through line [11] to cyclohexanone distillation column [F], where cyclohexanone is distilled overhead through line [12]. The bottom product is fed through line [13] to cyclohexanol distillation column [G], where a mixture comprising cyclohexanol and cyclohexanone is distilled overhead and passed through line [14] to cyclohexanol dehydrogenation unit [H]. Cyclohexanol dehydrogenation unit [H] comprises one or more cyclohexanol dehydrogenation reactors. The resulting mixture comprising cyclohexanone is, after separating of hydrogen gas (not shown in FIG. 2), recycled through line [16] to lights distillation column [E]. Optionally, this hydrogen gas is charged to hydrogenation unit [A] (not shown in FIG. 2). The bottom product of [G] comprising phenol is removed through line [15]. Line [15] leads to phenol distillation column [I] where a mixture comprising phenol is distilled overhead and fed through line [18] to first hydrogenation unit [A]. The bottom product is removed from phenol distillation column [I] through line [17].

According to one embodiment of the present invention, a chemical plant according to FIG. 2 is constructed from a chemical plant according to FIG. 1. From a comparison of FIG. 2 with FIG. 1, it can be seen that the following apparatus is disconnected from the chemical plant of FIG. 1 when constructing the chemical plant of FIG. 2: first hydrogenation unit [A] comprising one or more multi-tubular reactors and optionally hydrogen gas feed [2]. Further, the following equipment is connected to the first hydrogenation unit [A] disconnected from the chemical plant of FIG. 1 when constructing the chemical plant of FIG. 2: lights distillation column [E] together with input line [16] from cyclohexanol dehydrogenation unit [H] and output lines [10] and [11]; and cyclohexanone distillation column [F] together with output lines [12] and [13]; and cyclohexanol distillation column [G] together with output lines [14] and [15]; cyclohexanol dehydrogenation unit [H]; phenol distillation unit [I] together with output lines [17] and [18]. Output line [18] is connected to first hydrogenation unit [A]. Input line [7] is connected to the first hydrogenation unit [A]. Input line [8] (feed of hydrogen gas) is connected to the first hydrogenation unit [A]. Input line [8] may be identical to input line [2] of FIG. 1. Accordingly, input line [2] needs not to be replaced. The benzene hydrogenation catalyst inside the tubes of the more multi-tubular reactor(s) has to be replaced by phenol hydrogenation catalyst.

The present invention therefore also provides a process for the the production of a mixture comprising cyclohexanone and cyclohexanol by hydrogenation of phenol, said process comprising:
a) disconnecting a multi-tubular reactor that has been used for producing cyclohexane by the hydrogenation of benzene from a chemical plant, which plant was used for the production of a mixture comprising cyclohexanone and cyclohexanol by hydrogenation of benzene to form cyclohexane followed by oxidation of said cyclohexane;
b) replacing the benzene hydrogenation catalyst inside the tubes of said multi-tubular reactor with phenol hydrogenation catalyst; and
c) connecting said multi-tubular reactor to a chemical plant for the production of a mixture comprising cyclohexanone and cyclohexanol by hydrogenation of phenol; wherein "has been used" means that the multi-tubular reactor was designed for the hydrogenation of benzene and installed in a plant for the hydrogenation of benzene.

The present invention is illustrated by the following examples.

### COMPARATIVE EXPERIMENT A

A continuous process for the production of cyclohexane by hydrogenation of benzene was performed for a series of years in a chemical plant, comprising:
- a first hydrogenation unit;
- a second hydrogenation unit;
- a hydrogen separation unit;
as described above and substantially as depicted in FIG. 1. This commercial chemical plant had on average an hourly output of about 6.6 metric tons of essentially pure cyclohexane, which is equivalent to an annual plant output of approximately 53 kta of essentially pure cyclohexane (assuming 8000 effective production hours per year).

The hydrogenation of benzene in both the first and second hydrogenation unit was performed with 0.3 wt.% Pt/Al₂O₃ as catalyst. In the first hydrogenation unit the conversion of benzene into cyclohexane was already over 99 %. In the second hydrogenation unit most of the remaining benzene was converted.

The reaction mixture exiting the second hydrogenation unit was cooled down and hydrogen gas was separated from this reaction mixture and the resulting cyclohexane-comprising flow was used for the production of cyclohexanone via a cyclohexanone oxidation process.

The first hydrogenation unit contained one vertical multi-tubular reactor with benzene hydrogenation catalyst inside the tubes and had indirect cooling with a coolant for removal of heat. The vertical multi-tubular reactor had an internal diameter of about 2.2 m, containing 3528 tubes, each of which have a length of 4 m and an internal diameter of about 21.4 mm.

### EXAMPLE 1

In Example 1 (according to the invention), the vertical multi-tubular reactor in the first hydrogenation unit, was the same as the vertical multi-tubular reactor in the first hydrogenation unit in Comparative Experiment A.

A chemical plant for the production of cyclohexanone by hydrogenation of phenol, comprising:
- a first hydrogenation unit;
- a lights distillation column;
- a cyclohexanone distillation column;
- a cyclohexanol distillation column;
- a phenol distillation column; and
- a cyclohexanol dehydrogenation unit;
as described before and substantially as depicted in FIG. 2 was simulated in Aspen Plus® chemical engineering software. The simulated plant was designed with an identical vertical multi-tubular reactor in the first hydrogenation unit as in Comparative Experiment A. The vertical multi-tubular reactor in the first hydrogenation unit limited the overall capacity of the plant.

The hydrogenation of phenol in the phenol hydrogenation unit was performed in the gas phase in the presence of a phenol hydrogenation catalyst. The applied phenol hydrogenation catalyst was supplied by BASF (: 1 wt.% Pd on alumina support, with 1 wt.% Na (as NaHCO₃) added as promoter). The resulting gas mixture, comprising phenol, hydrogen gas, cyclohexanol and cyclohexanone, was partially condensed by cooling and separated into a liquid mixture comprising phenol, cyclohexanol and cyclohexanone that was fed to the lights distillation column, and a gaseous flow comprising hydrogen.

In the lights distillation column, components with boiling points lower than that of cyclohexanone were distilled overhead. The bottom flow from the lights distillation column was fed to the cyclohexanone distillation column, where essentially pure cyclohexanone was distilled overhead. The cyclohexanol concentration in the cyclohexanone that was distilled overhead in the cyclohexanone distillation column was on average about 500 ppm by weight. The bottom flow from the cyclohexanone distillation column was fed to the cyclohexanol distillation column, where a mixture comprising mainly cyclohexanol was distilled overhead. This mixture comprising mainly cyclohexanol was fed to the cyclohexanol dehydrogenation unit, in which cyclohexanol was converted into cyclohexanone. Hydrogen gas formed was separated therefrom. The resulting reaction mixture was then fed to the lights distillation column.

The bottom flow of the cyclohexanol distillation column was fed to a phenol distillation column where heavies were separated from a mixture comprising mainly cyclohexanol and phenol. In the cyclohexanol distillation column a mixture comprising phenol was distilled overhead and fed to the first hydrogenation unit.

Three months after start-up of the plant the following results could be obtained:
The gaseous feed rate charged to the vertical multi-tubular reactor in the first hydrogenation unit was about 15.3 ton/hr, comprising phenol, hydrogen, nitrogen, cyclohexanol, cyclohexanone and water. The phenol content of this gaseous feed was about 50 wt.%.

A gaseous mixture comprising cyclohexanone and cyclohexanol that was produced was discharged from the vertical multi-tubular reactor in the first hydrogenation unit at a temperature of 183 °C. This gaseous mixture also comprises phenol, 2-phenylcyclohexanol, 3-phenylcyclohexanol, 4-phenylcyclohexanol, cyclohexyl phenylether, benzofuran, 2,3-dimethylbenzofuran, 3-methyl-4-octanone, 4-methyl-3-octanone, 3-methyl-3-octanone, methyl-isopropylcyclohexanol, methyl-isopropylcyclohexanone, 1-(4-methylpentane-2-yl)-benzene-phenol, hydrogen and nitrogen. Analysis showed that more than 93 mol % of the phenol that was fed into the reactor was converted into cyclohexanone and cyclohexanol.

The hydrogenation rate of phenol was about (15.3 ton/hr * 50 wt.% * 93 mol % =) 7.1 ton/hr.

As coolant (liquid) water was fed to the vertical multi-tubular reactor in the first hydrogenation unit at a rate of about 4.7 ton/hr. Most of this water (more than 99%) left the vertical multi-tubular reactor in the first hydrogenation unit as steam (vapour).

As top product from the cyclohexanone distillation column about 7.4 ton/hr of essentially pure cyclohexanone (purity > 99.8 wt.%)was obtained.

Comparison of Comparative Experiment A and Example 1 shows that the vertical multi-tubular reactor in the first hydrogenation unit used in the production of cyclohexane by hydrogenation of benzene, can be re-used, after replacement of the catalyst, as a vertical multi-tubular reactor in the first hydrogenation unit for the production of a gaseous mixture comprising cyclohexanone and cyclohexanol by hydrogenation of phenol, and that from this mixture essentially pure cyclohexanone can be obtained.

## Claims

1. A continuous process for the production of a mixture comprising cyclohexanone and cyclohexanol by hydrogenation of phenol, which process is performed in an industrial scale chemical plant comprising a multi-tubular reactor **characterised in that** said multi-tubular reactor has been used for the hydrogenation of benzene; wherein the benzene hydrogenation catalyst inside the tubes of the multi-tubular reactor has been replaced with phenol hydrogenation catalyst;
and wherein "has been used" means that the multi-tubular reactor was designed for the hydrogenation of benzene and installed in a plant for the hydrogenation of benzene.

2. A process according to claim 1, wherein the internal diameter of the shell of said multi-tubular reactor is from 0.2 to 6 m.

3. A process according to claim 1 or claim 2, wherein the number of reactor tubes in said multi-tubular reactor is from 25 to 20,000.

4. A process according to any one of claims 1 to 3, wherein the internal diameter of the reactor tubes in said multi-tubular reactor is from 10 to 120 mm.

5. A process according to any one of claims 1 to 4, wherein the temperature of the mixture comprising cyclohexanone and cyclohexanol that is discharged from the multi-tubular reactor is from 100 to 220 °C.

6. A process according to any one of claims 1 to 5, wherein the hydrogenation is carried out in the presence of a supported palladium catalyst.

7. A process according to any one of claims 1 to 6, wherein water is applied as coolant in the multi-tubular reactor and more than 90 wt.% of the water is evaporated.

8. A process according to any one of claims 1 to 7, wherein a mixture comprising phenol and hydrogen, in which the phenol content is more than 25 wt.%, is charged as feed to the multi-tubular reactor.

9. A process according to any one of claims 1 to 8, wherein the molar ratio of cyclohexanone to cyclohexanol in the mixture comprising cyclohexanone and cyclohexanol that is produced is more than 4 to 1.

10. A process according to any one of claims 1 to 9, wherein the mixture comprising cyclohexanone and cyclohexanol that is produced also comprises phenol and at least one compound selected from 2-phenylcyclohexanol, 3-phenylcyclohexanol, 4-phenylcyclohexanol, cyclohexyl phenylether, benzofuran, 2,3-dimethylbenzofuran, 3-methyl-4-octanone, 4-methyl-3-octanone, 3-methyl-3-octanone, methyl-isopropylcyclohexanol, methyl-isopropylcyclohexanone and 1-(4-methylpentane-2-yl)-benzene-phenol.

11. A process according to any one of claims 1 to 10, wherein from the mixture comprising cyclohexanone and cyclohexanol that is produced, cyclohexanone with a purity of more than 99 wt.% is separated by distillation.

12. Use of an industrial scale chemical plant for the production of a mixture comprising cyclohexanone and cyclohexanol by hydrogenation of phenol, which chemical plant comprises a multi-tubular reactor **characterised in that** said multi-tubular reactor has been used for the hydrogenation of benzene;
wherein the benzene hydrogenation catalyst inside the tubes of the multi-tubular reactor has been replaced with phenol hydrogenation catalyst;
and wherein "has been used" means that the multi-tubular reactor was designed for the hydrogenation of benzene and installed in a plant for the hydrogenation of benzene.

13. The use of a chemical plant according to claim 12, which chemical plant further comprises a distillation column for the separation of cyclohexanone from a mixture of cyclohexanone and cyclohexanol with a purity of more than 99 wt.%.

14. A process for the construction of an industrial scale chemical plant for the production of a mixture comprising cyclohexanone and cyclohexanol by hydrogenation of phenol, said process comprising:
a) disconnecting a multi-tubular reactor that has been used for producing cyclohexane by the hydrogenation of benzene from a chemical plant, which plant was used for the production of a mixture comprising cyclohexanone and cyclohexanol by hydrogenation of benzene to form cyclohexane followed by oxidation of said cyclohexane;
b) replacing the benzene hydrogenation catalyst inside the tubes of said multi-tubular reactor with phenol hydrogenation catalyst; and
c) connecting said multi-tubular reactor to a chemical plant for the production of a mixture comprising cyclohexanone and cyclohexanol by hydrogenation of phenol;
wherein "has been used" means that the multi-tubular reactor was designed for the hydrogenation of benzene and installed in a plant for the hydrogenation of benzene.

## Patentansprüche

1. Ein kontinuierliches Verfahren zur Herstellung eines Cyclohexanon und Cyclohexanol umfassenden Gemischs durch die Hydrierung von Phenol, wobei dieses Verfahren im großtechnischen Maßstab in einer einen Mehrrohrreaktor umfassenden Chemieanlage durchgeführt wird, **dadurch gekennzeichnet, dass** dieser Mehrrohrreaktor vorher zur Hydrierung von Benzol verwendet wurde, wobei der Katalysator der Benzolhydrierung in den Rohren des Mehrrohrreaktors durch einen Katalysator der Phenolhydrierung ersetzt worden ist und "verwendet wurde" bedeutet, dass der Mehrrohrreaktor für die Benzolhydrierung konstruiert und in einer Chemieanlage zur Benzolhydrierung eingebaut war.

2. Ein Verfahren nach Anspruch 1, wobei der Innendurchmesser des Mantels des Mehrrohrreaktors 0,2 bis 6 m beträgt.

3. Ein Verfahren nach Anspruch 1 oder 2, wobei die Anzahl der Reaktorrohre in dem Mehrrohrreaktor 25 bis 20 000 beträgt.

4. Ein Verfahren nach einem der Ansprüche 1 bis 3, wobei der Innendurchmesser der Reaktorrohre in dem Mehrrohrreaktor 10 bis 120 mm beträgt.

5. Ein Verfahren nach einem der Ansprüche 1 bis 4, wobei die Temperatur des Cyclohexanon und Cyclohexanol umfassenden Gemischs, das den Mehrrohrreaktor verlässt, 100 bis 220 °C beträgt.

6. Ein Verfahren nach einem der Ansprüche 1 bis 5, wobei die Hydrierung in Gegenwart eines auf einem Träger befindlichen Palladiumkatalysators durchgeführt wird.

7. Ein Verfahren nach einem der Ansprüche 1 bis 6, wobei in dem Mehrrohrreaktor als Kühlmittel Wasser verwendet wird und mehr als 90 Gew.-% des Wassers verdampft ist.

8. Ein Verfahren nach einem der Ansprüche 1 bis 7, wobei ein Phenol und Wasserstoff umfassendes Gemisch, dessen Phenolgehalt mehr als 25 Gew.-% beträgt, als Zulauf in den Mehrrohrreaktor geleitet wird.

9. Ein Verfahren nach einem der Ansprüche 1 bis 8, wobei das Molverhältnis von Cyclohexanon zu Cyclohexanol in dem Cyclohexanon und Cyclohexanol umfassenden Gemisch, das hergestellt wird, mehr als 4 zu 1 beträgt.

10. Ein Verfahren nach einem der Ansprüche 1 bis 9, wobei das Cyclohexanon und Cyclohexanol umfassende Gemisch, das hergestellt wird, auch Phenol und mindestens eine Verbindung, die aus 2-Phenylcyclohexanol, 3-Phenylcyclohexanol, 4-Phenylcyclohexanol, Cyclohexylphenylether, Benzofuran, 2,3-Dimethylbenzofuran, 3-Methyl-4-octanon, 4-Methyl-3-octanon, 3-Methyl-3-octanon, Methylisopropylcyclohexanol, Methylisopropylcyclohexanon und 1-(4-Methylpentan-2-yl)-benzolphenol ausgewählt ist, enthält.

11. Ein Verfahren nach einem der Ansprüche 1 bis 10, wobei aus dem Cyclohexanon und Cyclohexanol umfassenden Gemisch, das hergestellt wird, Cyclohexanon mit einer Reinheit von über 99 Gew.-% durch Destillation abgetrennt wird.

12. Verwendung einer großtechnischen Chemieanlage zur Herstellung eines Cyclohexanon und Cyclohexanol umfassenden Gemischs durch die Hydrierung von Phenol, wobei die Chemieanlage einen Mehrrohrreaktor umfasst, **dadurch gekennzeichnet, dass** dieser Mehrrohrreaktor vorher zur Hydrierung von Benzol verwendet wurde, wobei der Katalysator der Benzolhydrierung in den Rohren des Mehrrohrreaktors durch einen Katalysator der Phenolhydrierung ersetzt worden ist und "verwendet wurde" bedeutet, dass der Mehrrohrreaktor für die Benzolhydrierung konstruiert und in einer Chemieanlage zur Benzolhydrierung eingebaut war.

13. Die Verwendung einer Chemieanlage nach Anspruch 12, wobei die Chemieanlage ferner eine Destillationskolonne, um aus einem Cyclohexanon-Cyclohexanol-Gemisch Cyclohexanon mit einer Reinheit von über 99 Gew.-% abzutrennen, umfasst.

14. Ein Verfahren zur Ausführung einer Chemieanlage im großtechnischen Maßstab für die Herstellung eines Cyclohexanon und Cyclohexanol umfassenden Gemischs durch die Hydrierung von Phenol, wobei dieses Verfahren das:
a) Trennen eines Mehrrohrreaktors, der für die Herstellung von Cyclohexan durch die Hydrierung von Benzol verwendet wurde, von einer Chemieanlage, wobei die Chemieanlage für die Herstellung eines Cyclohexanon und Cyclohexanol umfassenden Gemischs durch die Hydrierung von Benzol, wobei Cyclohexan gebildet und anschließend dieses Cyclohexan oxidiert wurde, verwendet wurde,
b) Austauschen des Katalysators der Benzolhydrierung in den Rohren dieses Mehrrohrreaktors gegen einen Katalysator der Phenolhydrierung und
c) Anschließen dieses Mehrrohrreaktors an eine Chemieanlage für die Herstellung eines Cyclohexanon und Cyclohexanol umfassenden Gemischs durch Hydrierung von Phenol
umfasst, wobei "verwendet wurde" bedeutet, dass der Mehrrohrreaktor für die Benzolhydrierung konstruiert und in einer Chemieanlage für die Benzolhydrierung eingebaut war.

## Revendications

1. Processus continu destiné à la production, par hydrogénation de phénol, d'un mélange comprenant de la cyclohexanone et du cyclohexanol, lequel processus est réalisé dans une usine chimique à échelle industrielle, ladite usine comprenant un réacteur multitubulaire **caractérisé en ce que** ledit réacteur multitubulaire a été utilisé pour l'hydrogénation de benzène ;
processus dans lequel le catalyseur d'hydrogénation de benzène disposé à l'intérieur des tubes du réacteur multitubulaire a été remplacé par un catalyseur d'hydrogénation de phénol ;
et processus dans lequel les termes "a été utilisé" signifient que le réacteur multitubulaire a été conçu pour l'hydrogénation de benzène et installé dans une usine prévue pour l'hydrogénation de benzène.

2. Processus selon la revendication 1, dans lequel le diamètre intérieur de la coque dudit réacteur multitubulaire est compris entre 0,2 m et 6 m.

3. Processus selon la revendication 1 ou la revendication 2, dans lequel le nombre de tubes du réacteur, disposés dans ledit réacteur multitubulaire, est compris entre 25 et 20 000.

4. Processus selon l'une quelconque des revendications 1 à 3, dans lequel le diamètre intérieur des tubes du réacteur, disposés dans ledit réacteur multitubulaire, est compris entre 10 mm et 120 mm.

5. Processus selon l'une quelconque des revendications 1 à 4, au cours duquel la température du mélange qui comprend de la cyclohexanone et du cyclohexanol et qui est déchargé du réacteur multitubulaire est entre 100°C et 220°C.

6. Processus selon l'une quelconque des revendications 1 à 5, au cours duquel l'hydrogénation est effectuée en présence d'un catalyseur au palladium disposé sur un support.

7. Processus selon l'une quelconque des revendications 1 à 6, au cours duquel de l'eau est appliquée comme liquide de refroidissement dans le réacteur multitubulaire, et plus de 90 % en poids de l'eau est évaporée.

8. Processus selon l'une quelconque des revendications 1 à 7, au cours duquel un mélange comprenant du phénol et de l'hydrogène, mélange dans lequel la teneur en phénol est de plus de 25 % en poids, est chargé comme alimentation fournie au réacteur multitubulaire.

9. Processus selon l'une quelconque des revendications 1 à 8, dans lequel le rapport molaire de cyclohexanone sur cyclohexanol, dans le mélange qui est produit et qui comprend de la cyclohexanone et du cyclohexanol, est de plus de 4 sur 1.

10. Processus selon l'une quelconque des revendications 1 à 9, dans lequel le mélange, qui est produit et qui comprend de la cyclohexanone et du cyclohexanol, comprend également du phénol et au moins un composé sélectionné parmi ceux tels que du 2-phénylcyclohexanol, du 3-phénylcyclohexanol, du 4-phénylcyclohexanol, de l'éther phénylique de cyclohexyle, du benzofurane, du 2,3-diméthyl-benzofurane, de la 3-méthyl-4-octanone, de la 4-méthyl-3-octanone, de la 3-méthyl-3-octanone, de l'isopropylcyclohexanol de méthyle, de l'isopropylcyclohexanone de méthyle et du 1-(4-méthylpentane-2-yl)-benzène-phénol.

11. Processus selon l'une quelconque des revendications 1 à 10, au cours duquel, à partir du mélange qui est produit et qui comprend de la cyclohexanone et du cyclohexanol, de la cyclohexanone ayant une pureté de plus de 99 % en poids est séparée par distillation.

12. Utilisation d'une usine chimique à échelle industrielle destinée à la production, par hydrogénation de phénol, d'un mélange comprenant de la cyclohexanone et du cyclohexanol, laquelle usine chimique comprend un réacteur multitubulaire **caractérisé en ce que** ledit réacteur multitubulaire a été utilisé pour l'hydrogénation de benzène ;
où le catalyseur d'hydrogénation de benzène disposé à l'intérieur des tubes du réacteur multitubulaire a été remplacé par un catalyseur d'hydrogénation de phénol ;
et où les termes "a été utilisé" signifient que le réacteur multitubulaire a été conçu pour l'hydrogénation de benzène et installé dans une usine prévue pour l'hydrogénation de benzène.

13. Utilisation d'une usine chimique selon la revendication 12, laquelle usine chimique comprend en outre une colonne de distillation prévue pour la séparation de cyclohexanone, à partir d'un mélange de cyclohexanone et de cyclohexanol, ladite cyclohexanone ayant une pureté de plus de 99 % en poids.

14. Processus pour la construction d'une usine chimique à échelle industrielle destinée à la production, par hydrogénation de phénol, d'un mélange comprenant de la cyclohexanone et du cyclohexanol, ledit processus consistant :
a) à déconnecter un réacteur multitubulaire qui a été utilisé pour produire du cyclohexane obtenu par l'hydrogénation de benzène réalisée par une usine chimique, laquelle usine a été utilisée pour la production, par hydrogénation de benzène, d'un mélange comprenant de la cyclohexanone et du cyclohexanol, pour former du cyclohexane, ladite formation étant suivie d'une oxydation dudit cyclohexane ;
b) à remplacer le catalyseur d'hydrogénation de benzène disposé à l'intérieur des tubes dudit réacteur multitubulaire, par un catalyseur d'hydrogénation de phénol ; et
c) à connecter ledit réacteur multitubulaire à une usine chimique destinée à la production, par hydrogénation de phénol, d'un mélange comprenant de la cyclohexanone et du cyclohexanol ;
processus dans lequel les termes "a été utilisé" signifient que le réacteur multitubulaire a été conçu pour l'hydrogénation de benzène et installé dans une usine prévue pour l'hydrogénation de benzène.
